# EUROPEAN PATENT SPECIFICATION

(11) **EP 1 901 788 B1**
(45) Date of publication and mention of the grant of the patent: **24.08.2011**
(21) Application number: 06755743.9
(22) Date of filing: 10.07.2006
(51) Int. Cl.: A61L 24/00, A61L 24/06, A61L 27/16, A61L 27/44, C08L 33/04

(54) **BONE CEMENT COMPOSITION**
KNOCHENZEMENTZUSAMMENSETZUNG
COMPOSITION DE CIMENT OSSEUX

(30) Priority: 08.07.2005 GB 0514076
(43) Date of publication of application: 26.03.2008
(73) Proprietor: Depuy International Limited, Beeston, Leeds LS11 8DT (GB)
(72) Inventor: BAKER, Diane, Lea, Preston PR2 1SA (GB); KOWALSKI, Rick, Preston PR4 0BY (GB)
(74) Representative: Belcher, Simon James
(86) International application number: PCT/GB2006/002532
(87) International publication number: WO 2007/007065

(56) References cited:
- EP-A1- 0 976 786
- WO-A-01/50974
- WO-A-81/02022
- WO-A-96/11714
- WO-A-98/30192
- WO-A-2007/007062
- US-A1- 2002 120 033
- DATABASE CAPLUS [Online] CHEMICAL ABSTRACTS SERVICE, COLUMBUS, OHIO, US; XP002423258 retrieved from STN Database accession no. 2004:1100210
- KENNY S M ET AL: "BONE CEMENTS AND FILLERS: A REVIEW" JOURNAL OF MATERIALS SCIENCE. MATERIALS IN MEDICINE, CHAPMAN AND HALL, LONDON, GB, vol. 14, 2003, pages 923-938, XP000962760 ISSN: 0957-4530

## Description

The present invention relates to formulations for bone cement compositions. Such compositions can be used in the treatment of bone defects or fractures arising both from procedures for treatment of conditions arising from natural degeneration and trauma, and in the fixation of prostheses and other implant articles.

The present invention therefore seeks to provide cement formulations for bonding to bone for use in surgical procedures.

A number of attempts have been carried out to provide bone cement which is mechanically stable in order to provide good fixation in orthopaedic applications, and which can stimulate a bone reaction which is consistent with strong fixation. Glass ionomer cements are used in dentistry but lack the mechanical strength for use in orthopaedic applications. Calcium phosphate cements can be seen sometimes as having the disadvantage that they are too weak to be used in weight-bearing indications without the use of pins or screws etc.

Cement formulations containing a high loading of tricalcium phosphate (TCP) in dimethacrylate monomer (bis-GMA) have been tried but suffer the disadvantage of high losses of tricalcium phosphate due to the hydrophilic nature of the monomer. The monomer causes the TCP to resorb or dissolve too quickly to allow good bone attachment. A further problem arises in that hydrophilic monomers such as Bis-GMA, urethane dimethacrylate monomer (UDMA) and tetrahydrofurfuryl methacrylate monomer (THFMA) can only be made into paste systems because they cannot attack sufficiently the polymer powder in order to form a dough. Consequently, cements made from these monomers do not have the desired handling characteristics for good cementation. It is also difficult to formulate a cement which has more that 50% (by dry weight) of inorganic material powder in the formulation because the handling characteristics are poor and the powder is very dry. Another problem arises in that it is difficult to sterilise paste systems.

WO-98/30192 discloses cement compositions which make use of acrylate monomers which are heterocyclic. The compositions can contain between 5 and 85% by weight of filler material. The filler material can include organic and inorganic materials, and can include inert materials such as alumina and glass particles.

The present invention provides a bone cement system which includes a liquid component and a powder component, in which the liquid component is formulated so that the properties arising from the constitution of the powder component can be optimised.

Accordingly, the invention provides a bone cement composition formed by mixing:
(a) a liquid formulation which comprises 5 to 35% by weight of a dimethacrylate monomer having a molecular weight of at least 250 and bearing at least one hydrophilic group, 5 to 35% by weight of a monofunctional methacrylate monomer having a molecular weight of not more than 250 bearing at least one hydrophilic group, 30 to 90% by weight of methyl methacrylate monomer, with
(b) a powder formulation which comprises (i) an inorganic filler which provides a source of calcium ions so that calcium ions can be leached from the cement composition when it has been implanted in a patient, the inorganic filler being present in the powder formulation in an amount of from 50 to 85% by weight of the powder formulation, (ii) one or more biocompatible polymers which can participate in polymerisation of the monomer components of the liquid formulation in order to form an interpenetrating network between the monomer components of the liquid formulation and the said biocompatible polymers, the biocompatible polymers having a molecular weight of at least 200,000 and being present in the powder formulation in an amount of from 15 to 50% by weight of the powder formulation, and (iii) an initiator in an amount of 0.10 to 2.0% by weight of the powder formulation,
in a ratio of from 1.9 to 2.9 parts by weight of powder to 1.0 part by weight of liquid.

Molecular weight values which are referred to in this specification are weight average molecular weights.

Preferably, the methacrylate monomer which makes up 30 to 90% by weight of the liquid formulation is methyl methacrylate.

Preferably, the liquid formulation includes an activator for a polymerisation reaction between monomer molecules.

Preferably, the powder formulation includes an initiator for a polymerisation reaction involving the biocompatible polymer. The initiator can be present in an amount of 0.10 to 2.0% by weight of the powder formulation.

A bone cement can be produced from the liquid and powder formulations mentioned above by a copolymerisation reaction between the polymerisable components of the liquid and powder formulations. The formation of cements by reactions of this kind is well known. The reaction can involve initiation by appropriate activators or initiators or both, as is known. For example, the reaction can be initiated by an initiator which can initiate the polymerisation reaction on contact with the liquid formulation. Preferred initiators include organic peroxides such as benzoyl peroxide. The initiator can be provided in the powder formulation or can be provided separately. When the initiator is provided in the powder formulation, it is preferably present in an amount of at least about 0.10% by weight of the powder formulation, more preferably at least about 0.20%. Preferably, the initiator is present in an amount of not more than about 2.0% by weight of the powder formulation, more preferably not more than about 1.0%.

Initiation of the reaction can involve use of an activator. Examples of suitable activators include certain tertiary amines, including N,N-dimethyl-p-toluidine or N,N-dihydroxyethyl-p-toluidine. When the polymerization reaction can then be initiated when the initiator and activator come into contact with one another during the physical mixing of the powder and liquid.

It can be preferred for the liquid formulation also to include an inhibitor to inhibit polymerisation of the monomer components. This can help to reduce the spontaneous polymerisation reaction which can be observed with certain monomers such as methacrylate monomers under normal storage conditions. Suitable inhibitors include hydroquinone, monomethylhydroquinone and butylated hydroxytoluene. The inhibitor can be present in an amount of 0.001 to 1.00% by weight.

The high molecular weight polymer in the powder formulation should be capable of participating in the polymerisation of polymerisable components of the liquid formulation in order to form an interpenetrating network between the polymerisable components of the liquid formulation and the high molecular weight polymer in the powder formulation. Preferably, the high molecular weight polymer in the powder formulation or the long chain monomer in the liquid formulation, or more preferably each of them, should be capable of participating in a cross-linking reaction. Cross-linking can add structural integrity to the cured cement.

Preferably, the high molecular weight polymer in the powder formulation is soluble in the liquid formulation when the liquid and powder formulations are mixed, for example so as to form a solution or a gel. The characteristics of the mixture are also affected by the presence of inorganic filler. The formation of a gel by dissolution of the powder in the liquid can help to provide good handling characteristics, for example by conferring dough-like or pasty characteristics to the mixed components.

A preferred long chain monomer in the liquid formulation should contain hydrophilic groups. Preferred long chain monomers include urethane dimethacrylate (UDMA), bis-glycol dimethacrylate (bis-GMA), polyethylene glycol dimethacrylate (PEGDMA).

A preferred short chain monomer should contain hydrophilic groups. An example of a preferred monofunctional methacrylate monomer includes tetrahydrofurfuryl methacrylate (THFMA).

Preferably, the methacrylate-based monomer of the liquid formulation is methyl methacrylate. Examples of hydrophilic groups present on the long and short chain monomers include oxygen-containing groups and nitrogen-containing groups. Specific groups include oxygen containing heterocyclic groups, hydroxyl groups and amine or amide groups.

Release of calcium ions can help to a bone reaction which is consistent with strong fixation. For example, it can help to stimulate the growth of bone tissue, in particular into pores in the bone cement which result from leaching of the calcium ions from the cement material after the material has cured. The incorporation of a filler which has these properties renders the composition "bioactive".

Preferred fillers include calcium phosphates (such as beta-tricalcium phosphate (β-TCP)), calcium sulphate, and bioglass compositions. It will generally be preferred that the calcium containing compound composition is chosen so that it resorbs slowly on the surface of the cement but does not resorb or dissolve out of the cement too quickly to prevent osteo-conduction from occurring. It is also important that degradation of the cement does not occur to an unacceptable extent.

The filler is present in the powder formulation in an amount of not more than 85%, especially not more than about 75% by weight of the powder formulation and in an amount of at least 50% by weight of the powder formulation

Ratio by weight of the powder and liquid formulations which are mixed to form the cement is from 1,9 to 2,9 parts by weight of powder to 10 part by weight of liquid. Preferably, the value of that ratio is not more than about 2.75, for example not more than about 2.57.

Preferably, the amount of the filler in the cement composition is at least 10% by weight, based on the total weight of the composition, more preferably at least 25%, especially at least 40%, for example at least 50% or at least 51%. Preferably, the amount of the filler in the cement composition is not more than 70% by weight, based on the total weight of the composition, more preferably not more than 65%, especially not more than 63%, for example not more than 55%.

Preferably, the inorganic filler has a median particle size measured using a Beckman Coulter laser diffraction particle size analyser which is at least about 1 µm, more preferably at least about 3 µm. Preferably, the size of the inorganic filler particles is not more than about 20 µm, more preferably not more than about 15 µm.

The high molecular weight polymer in the powder may be a single polymer or a combination of polymers. It can help to modify the viscosity of the material of the anchor layer in the period before it hardens.

In this embodiment in order to achieve dough-like consistency viscous monomers are used together with the polymerisable components in the liquid formulation and a high molecular weight polymer in the powder formulation. Suitable polymers include poly(methyl methacrylate), copolymers of methyl methacrylate. Suitable copolymers are made from monomers by copolymerisation with methyl methacrylate and such monomers include styrene, ethyl methacrylate, butyl methacrylate and methyl acrylate. Methyl acrylate-methylmethacrylate copolymer is the preferred copolymer.

Preferably, the molecular weight (number average) of the high molecular weight polymer in the powder formulation is at least

about 500,000, and most preferably is at least about 750,000. The high molecular weight polymer is present in the powder formulation in an amount of at least 15% by weight of the powder formulation, more preferably at least 25%, especially at least 40%. The high molecular weight polymer is present in the powder formulation in an amount of not more than 85% more preferably not more than 80% especially not more than 70%.

Preferably, the resulting bone cement has a compressive strength measured by the method of ISO 5833 of at least 70 MPa.

The use of a filler which can act as a source of calcium ions has the advantage of stimulating bone growth, at least under favourable conditions.

The use of the combination of monomers in the liquid formulation, in conjunction with the polymer component in the powder formulation, can help to confer useful handling characteristics on the cement composition, notwithstanding the presence of a high loading of an inorganic filler material. Accordingly, the cement composition can have the consistency of a dough rather than a paste. This allows the handling characteristics of the cement of the present invention to be compatible with current surgical cementation techniques. This can be contrasted with known cement compositions with a high proportion of filler (for example, 50% by weight or more) which have tended to have paste-like consistencies.

The use of high inorganic filler loadings in the cement is facilitated by use of the monomer mixture used in the liquid component. The monomer mixture of the present invention ensures that the dispersion of the filler which is formed on mixing the powder and liquid is homogenous. A disadvantage of prior art compositions is that the dispersion of the filler formed on mixing may not be homogenous. Without wishing to be bound by theory, it is believed that the hydrophilic properties arising as a blend of the monomers used in the present invention assist in controlling the dispersion of the hydrated filler. It is also believed that the liquid monomer mixture can have an effect on the elution of calcium from the filler and also on the water uptake. The degree to which these effects are manifested may be controlled by varying the total monomer quantity in the cement composition and also the composition of the liquid.

The bone cement composition of the present invention addresses some or all of the problems of the prior art. In particular, known bone cements commonly comprise an inorganic powder filler and a curable liquid monomer. If the resulting cured cement is too hydrophilic then a water soluble filler such as calcium phosphate will be resorbed or dissolved too quickly for good long term fixation to bone tissue. The compositions of the present invention can help to minimise this problem.

The bone cement composition of the invention can be used in the treatment of bone defects, and in the treatment of fractures. It can be used in the treatment of conditions arising from natural degeneration and trauma. The composition of the invention finds particular application in the fixation of prostheses and other implant articles.

The properties of the cement composition of the invention can be used to advantage in other applications. For example, the ability of the composition to stimulate the growth of bone tissue, in particular into voids in the bone cement which result from leaching of the calcium ions from close to the surface of the cement material after the material has cured, means that the cement composition can be used, after it has been cured, as an implant material. The properties of the material can then facilitate fixation of the implant. For example, the composition of the invention can be used to make an implant which is to be placed in contact with a patient's bone to fill a defect in the patient's natural tissue. This might be a defect in a bone. The material can be used in an anchor layer of an implant, for example for use in repair to cartilage defect.

The present invention will now be illustrated by the following examples.

### EXAMPLE 1

A powder formulation was produced containing:

| Constituent | % w/w |
|---|---|
| β-TCP | 79.75 |
| Benzoyl peroxide | 0.25 |
| Methyl acrylate-methyl methacrylate copolymer | 20.00 |

A liquid formulation was produced containing:

| Constituent | % w/w |
|---|---|
| Methylmethacrylate monomer | 66.0 |
| Urethane dimethacrylate monomer | 16.5 |
| Tetrahydrofurfuryl methacrylate monomer | 16.5 |
| Dimethyl-p-toluidine | 1.0 |
| Hydroquinone | 50 ppm |

The powder formulation was mixed with the liquid formulation in the ratio 2.13 parts by weight powder to 1 part by weight liquid formulation.

### EXAMPLE 2

A powder component was produced containing:

| Constituent | % w/w |
|---|---|
| β-TCP | 70.0 |
| Benzoyl peroxide | 6.60 |
| Methyl acrylate-methyl methacrylate copolymer | 29.40 |

A liquid formulation was produced containing:

| Constituent | % w/w |
|---|---|
| Methylmethacrylate monomer | 66.0 |
| Urethane dimethacrylate monomer | 16.5 |
| Tetrahydrofurfuryl methacrylate monomer | 16.5 |
| Dimethyl-p-toluidine | 1.0 |
| Hydroquinone | 50 ppm |

The powder formulation was mixed with the liquid formulation in the ratio 2.33 parts by weight powder to 1 part by weight liquid formulation.

### EXAMPLE 3

A powder component was produced containing:

| Constituent | % w/w |
|---|---|
| β-TCP | 79.5 |
| 33% benzoyl peroxide in dicalcium phosphate | 0.8 |
| Methyl acrylate-methyl methacrylate copolymer | 19.7 |

A liquid formulation was produced containing:

| Constituent | % w/w |
|---|---|
| Methylmethacrylate monomer | 66.0 |
| Urethane dimethacrylate monomer | 16.5 |
| Tetrahydrofurfuryl methacrylate monomer | 16.5 |
| Dimethyl-p-toluidine | 1.0 |
| Hydroquinone | 50 ppm |

The powder formulation was mixed with the liquid formulation in the ratio 2.0 parts by weight powder to 1.0 part by weight liquid formulation.

### RESULTS

The compositions of the present invention have been evaluated *in vivo.* Eight week old Wistar rats weighing 180 to 230 grams were used in an implantation study. The rats were operated on under general anaesthesia introduced by intraperitoneal injection of sodium-5-ethyl-5-(1-methylbutyl) barbiturate (known as Nembutal and obtainable from Dainippon Pharmaceutical Co, Osaka, Japan) at 40 mg/kg body weight. Cortical bone defects measuring 2 x 7 mm were introduced into each of the rats at the medial aspect of the proximal metaphysis of both tibiae, and bone marrow was curetted. The intramedullary canals of both bone defects were irrigated with physiological saline and a paste-form cement was inserted at random.

Each paste was allowed to cure *in situ* for evaluation of osteoconductivity. A total of 30 rats were evaluated, with one leg for the composition of Example 3 and one leg for the CMW1 composition. CMW1 is commercially available polymethyl methacrylate bone cement available from DePuy International Limited and was used as a control. Six rats were sacrificed respectively at 4, 8, 12, 26 and 52 weeks after the operation.

The powder of Example 3 was composed of methyl methacrylate-methyl acrylate copolymer, benzoyl peroxide in inert dicalcium phosphate base and β-TCP. The average particle size of the β-TCP was 8 µm. The liquid of Example 3 was composed of methyl methacrylate monomer, urethane dimethacrylate monomer, tetrahydrofurfuryl methacrylate monomer and dimethyl-p-toluidine and hydroquinone. The compositions of the powder and liquid are shown above in Example 3. The weight ratio of powder to liquid was approximately 2.25. The β-TCP was present in an amount of over 50% (approximately 53%) by weight of the total weight of the cement in Example 3.

The cement formed from the powder and liquid formulations of Example 3 was able to contact the bone directly without intervening soft tissue in the specimens at each time interval. Contact micro radiography revealed that this cement contacted the bone directly. There was always a soft tissue layer between CMW1 and bone. It was also revealed that the invasion of bony tissue into marginal cement of Example 3 was observed within 4 weeks and deeper invasion was observed at longer time intervals.

Histological findings demonstrate that the cement of Example 3 was biocompatible and the bony tissue invasion into cement of Example 3 seen in scanning electron microscope photographs indicated no toxic effect of the cement component.

It was also demonstrated that this cement was an excellent osteoconductive material as over half of the cement margin contacted directly to bone within 4 weeks of implantation and a larger part did by 26 weeks after implantation. The cement of Example 3 thus demonstrated a clear advantage over the sample CMW1.

## Claims

1. A bone cement composition formed by mixing:
(a) a liquid formulation which comprises 5 to 35% by weight of a dimethacrylate monomer having a molecular weight of at least 250 and bearing at least one hydrophilic group, 5 to 35% by weight of a monofunctional methacrylate monomer having a molecular weight of not more than 250 bearing at least one hydrophilic group, 30 to 90% by weight of methyl methacrylate monomer, with
(b) a powder formulation which comprises (i) an inorganic filler which provides a source of calcium ions so that calcium ions can be leached from the cement composition when it has been implanted in a patient, the inorganic filler being present in the powder formulation in an amount of from 50 to 85% by weight of the powder formulation, (ii) one or more biocompatible polymers which can participate in polymerisation of the monomer components of the liquid formulation in order to form an interpenetrating network between the monomer components of the liquid formulation and the said biocompatible polymers, the biocompatible polymers having a molecular weight of at least 200,000 and being present in the powder formulation in an amount of from 15 to 50% by weight of the powder formulation, and (iii) an initiator in an amount of 0.10 to 2.0% by weight of the powder formulation,
in a ratio of from 1.9 to 2.9 parts by weight of powder to 1.0 part by weight of liquid.

2. A bone cement composition as claimed in claim 1, wherein the high molecular weight monomer is selected from the group comprising urethane dimethacrylate (UDMA), polyethylene glycol dimethacrylate (PEGDMA) and bisglycol dimethacrylate (bis-GMA).

3. A bone cement composition as claimed in claim 1, wherein the low molecular weight monomer is a hydrophilic monomer which is not capable of cross-linking.

4. A bone cement composition as claimed in claim 1, in which the low molecular weight monomer is tetrahydrofurfuryl methacrylate (THFMA).

5. A bone cement composition as claimed in claim 1, in which the hydrophilic groups are oxygen-containing and/or nitrogen-containing groups.

6. A bone cement composition as claimed in claim 1, which includes an inhibitor in an amount of 0.001 to 1.00% by weight which inhibits polymerisation of polymerisable components of the liquid formulation.

7. A bone cement composition as claimed in claim 1, in which the inorganic filler is a calcium containing compound.

8. A bone cement composition as claimed in claim 7, in which the calcium containing compound is selected from the group comprising: calcium phosphate (e.g. beta-tricalcium phosphate), calcium sulphate and bioglass.

9. A bone cement composition as claimed in claim 1, in which the initiator is an organic peroxide.

10. A bone cement composition as claimed in claim 1, in which the inorganic filler is present in an amount of at least about 50% by weight, based on the total weight of the cement composition.

## Patentansprüche

1. Knochenzementzusammensetzung, gebildet durch Mischen:
(a) einer flüssigen Formulierung, die 5 bis 35 Gew.-% eines Dimethacrylat-Monomers, das ein Molekulargewicht von wenigstens 250 besitzt und wenigstens eine hydrophile Gruppe trägt, 5 bis 35 Gew.-% eines monofunktionellen Methacrylat-Monomers, das ein Molekulargewicht von nicht mehr als 250 besitzt, das wenigstens eine hydrophile Gruppe trägt, 30 bis 90 Gew.-% Methylmethacrylat-Monomer umfasst, mit
(b) einer Pulverformulierung, die (i) einen anorganischen Füllstoff, der eine Quelle für Calcium-Ionen bereitstellt, so dass Calcium-Ionen aus der Zementzusammensetzung ausgelaugt werden können, wenn sie in einem Patienten implantiert worden ist, wobei der anorganische Füllstoff in der Pulverformulierung in einer Menge von 50 bis 85 Gew.-% der Pulverformulierung vorliegt, (ii) eine oder mehrere biokompatible Polymere, die an der Polymerisation der Monomer-Komponenten der flüssigen Formulierung teilnehmen können, um ein interpenetrierendes Netzwerk zwischen den Monomer-Komponenten der flüssigen Formulierung und den besagten biokompatiblen Polymeren zu bilden, wobei die biokompatiblen Polymere ein Molekulargewicht von wenigstens 200.000 besitzen und in der Pulverformulierung in einer Menge von 15 bis 50 Gew.-% der Pulverformulierung vorliegen, und (iii) einen Initiator in einer Menge von 0,10 bis 2,0 Gew.-% der Pulverformulierung umfasst,
in einem Verhältnis von 1,9 bis 2,9 Gewichtsteilen Pulver zu 1,0 Gewichtsteil Flüssigkeit.

2. Knochenzementzusammensetzung nach Anspruch 1, wobei das Monomer mit hohem Molekulargewicht ausgewählt ist aus der Gruppe, bestehend aus Urethandimethacrylat (UDMA), Polyethylenglykoldimethacrylat (PEGDMA) und Bisglykoldimethacrylat (bis-GMA).

3. Knochenzementzusammensetzung nach Anspruch 1, wobei das Monomer mit niedrigem Molekulargewicht ein hydrophiles Monomer ist, das nicht zu Vernetzung in der Lage ist.

4. Knochenzementzusammensetzung nach Anspruch 1, in der das Monomer mit niedrigem Molekulargewicht Tetrahydrofurfurylmethacrylat (THFMA) ist.

5. Knochenzementzusammensetzung nach Anspruch 1, in der die hydrophilen Gruppen sauerstoffhaltige und/oder stickstoffhaltige Gruppen sind.

6. Knochenzementzusammensetzung nach Anspruch 1, die einen Hemmer in einer Menge von 0,001 bis 1,00 Gew.-% einschließt, der die Polymerisation polymerisierbarer Komponenten der flüssigen Formulierung hemmt.

7. Knochenzementzusammensetzung nach Anspruch 1, in der der organische Füllstoff eine calciumhaltige Verbindung ist.

8. Knochenzementzusammensetzung nach Anspruch 7, in der die calciumhaltige Verbindung ausgewählt ist aus der Gruppe, die: Calciumphosphat (z.B. beta-Tricalciumphosphat), Calciumsulfat und Bioglas umfasst.

9. Knochenzementzusammensetzung nach Anspruch 1, in der der Initiator ein organisches Peroxid ist.

10. Knochenzementzusammensetzung nach Anspruch 1, in der der organische Füllstoff in einer Menge von wenigstens etwa 50 Gew.-%, bezogen auf das Gesamtgewicht der Zementzusammensetzung, vorliegt.

## Revendications

1. Composition de ciment osseux formée par mélange :
(a) d'une formulation liquide comprenant de 5 à 35 % en poids d'un monomère de diméthacrylate possédant un poids moléculaire d'au moins 250 et comprenant au moins un groupe hydrophile, de 5 à 35 % en poids d'un monomère de méthacrylate monofonctionnel possédant un poids moléculaire de pas plus de 250 et comprenant au moins un groupe hydrophile, de 30 à 90 % en poids d'un monomère de méthacrylate de méthyle, avec
(b) une formulation de poudre qui comprend (i) une charge inorganique qui offre une source d'ions calcium de sorte que les ions calcium puissent être lixiviés de la composition de ciment lorsqu'elle a été implantée dans un patient, la charge inorganique étant présente dans la formulation de poudre en une quantité de 50 à 85 % en poids de la formulation de poudre, (ii) un ou plusieurs polymères biocompatibles qui peuvent participer à la polymérisation des composants monomères de la formulation liquide afin de former un réseau d'interpénétration entre les composants monomères de la formulation liquide et lesdits polymères biocompatibles, les polymères biocompatibles possédant un poids moléculaire d'au moins 200 000 et étant présents dans la formulation de poudre en une quantité de 15 à 50 % en poids de la formulation de poudre, et (iii) un initiateur en une quantité de 0,10 à 2,0 % en poids de la formulation de poudre,
selon un rapport de 1,9 à 2,9 parties en poids de poudre pour 1,0 partie en poids de liquide.

2. Composition de ciment osseux selon la revendication 1, dans laquelle le monomère de poids moléculaire élevé est choisi dans le groupe comprenant le diméthacrylate d'uréthane (UDMA), le diméthacrylate de polyéthylène glycol (PEGDMA) et le diméthacrylate de bisglycol (bis-GMA).

3. Composition de ciment osseux selon la revendication 1, dans laquelle le monomère de poids moléculaire faible est un monomère hydrophile qui n'est pas capable de réticulation.

4. Composition de ciment osseux selon la revendication 1, dans laquelle le monomère de poids moléculaire faible est le méthacrylate de tétrahydrofurfuryle (THFMA).

5. Composition de ciment osseux selon la revendication 1, dans laquelle les groupes hydrophiles sont des groupes contenant de l'oxygène et/ou contenant de l'azote.

6. Composition de ciment osseux selon la revendication 1, qui comprend un inhibiteur en une quantité de 0,001 à 1,00 % en poids qui inhibe la polymérisation des composants polymérisables de la formulation liquide.

7. Composition de ciment osseux selon la revendication 1, dans laquelle la charge inorganique est un composé contenant du calcium.

8. Composition de ciment osseux selon la revendication 7, dans laquelle le composé contenant du calcium est choisi dans le groupe comprenant le phosphate de calcium (par exemple le bêta-phosphate tricalcique), le sulfate de calcium et un bioverre.

9. Composition de ciment osseux selon la revendication 1, dans laquelle l'initiateur est un peroxyde organique.

10. Composition de ciment osseux selon la revendication 1, dans laquelle la charge inorganique est présente en une quantité d'au moins environ 50 % en poids, sur la base du poids total de la composition de ciment.
